# EUROPEAN PATENT APPLICATION

(11) **EP 4 768 908 A1**
(43) Date of publication of application: **01.07.2026**
(21) Application number: 25223955.3
(22) Date of filing: 16.12.2025
(51) Int. Cl.: G01N 33/00

(54) **METHODS, APPARATUSES, AND SYSTEMS FOR GAS SENSOR DRIFT ADJUSTMENT**

(30) Priority: 20.12.2024 US 202463737360 P; 11.12.2025 US 202519416803
(71) Applicant: Honeywell International Inc., Charlotte, NC 28202 (US)
(72) Inventor: MARSH, Brian J., Charlotte, 28202 (US); TIMMS, Jarod, Charlotte, 28202 (US); STOLFUS, Joel D., Charlotte, 28202 (US); OLSON, Thomas J., Charlotte, 28202 (US)
(74) Representative: Haseltine Lake Kempner LLP

(57) **Abstract**

Embodiments of the present disclosure provide techniques for sensor drift adjustment. A plurality of sensor measurements may be received from a gas sensor for at least a portion of a sampling period. Sampled sensor data may be generated from the plurality of sensor measurements. The sampled sensor data may comprise at least a subset of the plurality of sensor measurements that satisfy a set of operational conditions and a sample size threshold. Estimated drift data may be generated based on the sampled sensor data and one or more drift constraint. The estimated drift data may be stored. The gas sensor output may be adjusted by applying a drift adjustment model to the gas sensor output.

## Description

### CROSS REFERENCE TO RELATED APPLICATIONS

This application claims priority to U.S. Provisional Patent Application No. 63/737,360, filed on December 20, 2024, each of which is incorporated herein by reference in its entirety.

### TECHNICAL FIELD

The present disclosure relates to gas sensors and, more particularly, to methods, apparatuses, and systems for gas sensor drift adjustment.

### BACKGROUND

Applicant has identified many technical challenges and difficulties associated with gas sensor readings. Through applied effort, ingenuity, and innovation, Applicant has solved many of these identified problems by developing the embodiments of the present disclosure, which are described in detail below.

### BRIEF SUMMARY

In accordance with one aspect of the present disclosure, a computer-implemented method is provided. In some example embodiments, the method includes receiving a plurality of sensor measurements from a gas sensor for at least a portion of a sampling period; generating sampled sensor data from the plurality of sensor measurements, wherein the sampled sensor data comprises at least a subset of the plurality of sensor measurements that satisfy a set of operational conditions and a sample size threshold; generating estimated drift data based on the sampled sensor data and one or more drift constraints; storing the estimated drift data; and adjusting gas sensor output by applying a drift adjustment model to the gas sensor output.

In some embodiments, generating the sampled sensor data further includes generating variation data for the sampling period by applying one or more analytical models to the sampled sensor data; and determining if the variation data satisfies a variation threshold.

In some embodiments, the set of operational conditions comprise one or more of a temperature range, a humidity range, or a pressure stability duration.

In some embodiments, the method further includes receiving, from the gas sensor (i) temperature data, (ii) humidity data, and (iii) pressure data associated with a sensor measurement from the plurality of sensor measurements; and determining if (i) the temperature data satisfies a temperature threshold, (ii) the humidity data satisfies a humidity threshold; and (iii) the pressure data satisfies a pressure threshold.

In some embodiments, the gas sensor is configured for measuring hydrogen gas concentration in air.

In some embodiments, generating the estimated drift data further comprises generating the estimated drift data based one or more sensor output compensation constraints.

In some embodiments, receiving the plurality of sensor measurements comprises receiving each sensor measurement of the plurality of sensor measurements at a predetermined interval.

In accordance with another aspect of the present disclosure, a computer-implemented method is provided., an apparatus is provided. In some example embodiments, the apparatus comprises at least one processor and at least one memory storing instructions that, when executed by the at least one processor, cause the apparatus to receive a plurality of sensor measurements from a gas sensor for at least a portion of a sampling period; generate sampled sensor data from the plurality of sensor measurements, wherein the sampled sensor data comprises at least a subset of the plurality of sensor measurements that satisfy a set of operational conditions and a sample size threshold; generate estimated drift data based on the sampled sensor data and one or more drift rate constraints; store the estimated drift data; and adjust subsequent gas sensor output by applying a drift adjustment model to the gas sensor output.

In some embodiments, generating the sampled sensor data further comprises generating variation data for the sampling period by applying one or more analytical models to the sampled sensor data; and determining if the variation data satisfies a variation threshold.

In some embodiments, the set of operational conditions comprise one or more of a temperature range, a humidity range, or a pressure stability duration.

In some embodiments, the method further include receiving, from the gas sensor (i) temperature data, (ii) humidity data, and (iii) pressure data associated with a sensor measurement from the plurality of sensor measurements; and determining if (i) the temperature data satisfies a temperature threshold, (ii) the humidity data satisfies a humidity threshold; and (iii) the pressure data satisfies a pressure threshold.

In some embodiments, the gas sensor is configured for measuring hydrogen gas concentration in air.

In some embodiments, generating the estimated drift data further comprises generating the estimated drift data based one or more sensor output compensation constraints.

In some embodiments receiving the plurality of sensor measurements comprises receiving each sensor measurement of the plurality of sensor measurements at a predetermined interval.

In accordance with another aspect of the present disclosure, at least one non-transitory computer-readable storage medium is provided. In some example embodiments, the at least one non-transitory computer-readable storage medium includes computer coded instructions configured to, when executed by at least one processor receive a plurality of sensor measurements from a gas sensor for at least a portion of a sampling period; generate sampled sensor data from the plurality of sensor measurements, wherein the sampled sensor data comprises at least a subset of the plurality of sensor measurements that satisfy a set of operational conditions and a sample size threshold; generate estimated drift data based on the sampled sensor data and one or more drift rate constraints; store the estimated drift data; and adjust subsequent gas sensor output by applying a drift adjustment model to the gas sensor output.

In some embodiments, generating the sampled sensor data further comprises generating variation data for the sampling period by applying one or more analytical models to the sampled sensor data; and determining if the variation data satisfies a variation threshold.

In some embodiments, the set of operational conditions comprise one or more of a temperature range, a humidity range, or a pressure stability duration.

In some embodiments, the at least one non-transitory computer-readable storage medium further includes receiving, from the gas sensor (i) temperature data, (ii) humidity data, and (iii) pressure data associated with a sensor measurement from the plurality of sensor measurements; and determining if (i) the temperature data satisfies a temperature threshold, (ii) the humidity data satisfies a humidity threshold; and (iii) the pressure data satisfies a pressure threshold.

In some embodiments, the gas sensor is configured for measuring hydrogen gas concentration in air.

In some embodiments, receiving the plurality of sensor measurements comprises receiving each sensor measurement of the plurality of sensor measurements at a predetermined interval.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1A illustrates an example sensor device in accordance with at least one embodiment of the present disclosure.
FIG. 1B illustrates a block diagram of an example system architecture for a sensor in accordance with at least one embodiment of the present disclosure.
FIG. 2 illustrates a block diagram of an example apparatus that may be specially configured in accordance with at least one example embodiment of the present disclosure
FIG. 3 illustrates a cross-section view of an example thermal sensitive component in accordance with at least one embodiments of the present disclosure.
FIG. 4 illustrates an example flow chart diagram of an example process for sensor drift adjustment in accordance with at least one embodiment of the present disclosure.
FIG. 5 illustrates an example flow chart diagram of an example process for sensor drift measurement in accordance with at least one embodiment of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

Terms such as "computing," "determining," "generating," and/or similar words are used herein interchangeably to refer to the creation, modification, or identification of data. Further, "based on," "based on in part on," "based at least on," "based upon," and/or similar words are used herein interchangeably in an open-ended manner such that they do not indicate being based only on or based solely on the referenced element or elements unless so indicated. Like numbers refer to like elements throughout.

As used herein, terms such as "front," "rear," "top," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

As used herein, the term "or" is used in both the alternative and conjunctive sense, unless otherwise indicated. The terms "illustrative" and "example" are used to be examples with no indication of quality level. Terms such as "computing," "determining," "generating," and/or similar words are used herein interchangeably to refer to the creation, modification, or identification of data. Further, "based on," "based on in part on," "based at least on," "based upon," and/or similar words are used herein interchangeably in an open-ended manner such that they do not indicate being based only on or based solely on the referenced element or elements unless so indicated. Like numbers refer to like elements throughout.

### OVERVIEW AND TECHNICAL IMPROVEMENTS

A gas sensor is a device that may detect the presence and/or concentration level of a gas (e.g., a gaseous substance), including, for example, combustible gas, flammable gas, and/or toxic gas. For example, a gas sensor such as hydrogen leak detector sensor may be configured to detect the presence and/or concentration level of hydrogen (H2).

Many factors may affect the accuracies of gas sensor readings, including, for example, mechanical shock, wear and tear of components through use, exposure to high temperatures for a long period of time, change in properties of components within the gas, and/or the like. In particular, many gas sensors use sensing technology that is subject to long term drift errors. This drift error negatively affects the accuracy, particularly, long term accuracy, of the gas sensors. In some applications, the rate of change is substantially constant, which affects the accuracy of the sensor. Moreover, in many applications, a gas sensor may be in use for several years such that it is difficult or impossible to remove the gas sensor from the applications to perform re-calibrating, nulling, or zeroing of the sensor drift.

According to example embodiments herein, a drift adjustment technique may be employed to address the above problems. According to example embodiments, the drift adjustment technique is configured to detect if and when drift is happening, and correct for the drift (or facilitate correction of the drift) on an ongoing basis.

The drift adjustment techniques describes herein may be utilized in various sensors and applications. One non-limiting example, is a fuel cell powered vehicles or applications that includes stored hydrogen, piping from a hydrogen tank, and/or a fuel cell that is powered by hydrogen. These area are generally subject to hydrogen leakage. In this regard, in such applications, a gas sensor may be placed in the vicinity of these areas (e.g., storage tank, piping, and/or the fuel cell), and used to detect if there is a hydrogen leak occurring in these areas. For example, the sensor output (e.g., the primary sensor output) may be the hydrogen (H2) parts per million (PPM level). In such applications, a drift error may refer to a condition where the sensor output (e.g., H2 ppm output) from the gas sensor is starting to drift lower and lower over time. For example, when a sensor is first built, its sensor output would be zero H2 ppm in air with no hydrogen in it (e.g., no appreciable hydrogen). However, when installed in in a vehicle or an application and is exposed to a high temperature (e.g., 85°C, or the like) for a long period of time (e.g., 1000 hours), the sensor output is likely to drift from zero H2 ppm down to -300 H 2 ppm during the noted period. The drift adjustment techniques described herein is configured to re-zero out the drift (e.g., -300 H 2 ppm change in sensor output) back to its correct value (e.g., 0 H2 ppm in air).

### EXAMPLE SYSTEMS AND APPARATUSES OF THE DISCLOSURE

Embodiments of the present disclosure may be implemented in various ways, including as computer program products that comprise articles of manufacture. Such computer program products may include one or more software components including, for example, software objects, methods, data structures, or the like. A software component may be coded in any of a variety of programming languages. An illustrative programming language may be a lower-level programming language such as an assembly language associated with a particular hardware architecture and/or operating system platform. A software component comprising assembly language instructions may require conversion into executable machine code by an assembler prior to execution by the hardware architecture and/or platform. Another example programming language may be a higher-level programming language that may be portable across multiple architectures. A software component comprising higher-level programming language instructions may require conversion to an intermediate representation by an interpreter or a compiler prior to execution.

Other examples of programming languages include, but are not limited to, a macro language, a shell or command language, a job control language, a script language, a database query or search language, and/or a report writing language. In one or more example embodiments, a software component comprising instructions in one of the foregoing examples of programming languages may be executed directly by an operating system or other software component without having to be first transformed into another form. A software component may be stored as a file or other data storage construct. Software components of a similar type or functionally related may be stored together such as, for example, in a particular directory, folder, or library. Software components may be static (e.g., pre-established, or fixed) or dynamic (e.g., created or modified at the time of execution).

A computer program product may include a non-transitory computer-readable storage medium storing applications, programs, program modules, scripts, source code, program code, object code, byte code, compiled code, interpreted code, machine code, executable instructions, and/or the like (also referred to herein as executable instructions, instructions for execution, computer program products, program code, and/or similar terms used herein interchangeably). Such non-transitory computer-readable storage media include all computer-readable media (including volatile and non-volatile media).

A non-volatile computer-readable storage medium may include a floppy disk, flexible disk, hard disk, solid-state storage (SSS) (e.g., a solid-state drive (SSD), solid-state card (SSC), solid-state module (SSM)), enterprise flash drive, magnetic tape, or any other non-transitory magnetic medium, and/or the like. A non-volatile computer-readable storage medium may also include a punch card, paper tape, optical mark sheet (or any other physical medium with patterns of holes or other optically recognizable indicia), compact disc read only memory (CD-ROM), compact disc-rewritable (CD-RW), digital versatile disc (DVD), Blu-ray disc (BD), any other non-transitory optical medium, and/or the like. Such a non-volatile computer-readable storage medium may also include read-only memory (ROM), programmable read-only memory (PROM), erasable programmable read-only memory (EPROM), electrically erasable programmable read-only memory (EEPROM), flash memory (e.g., Serial, NAND, NOR, and/or the like), multimedia memory cards (MMC), secure digital (SD) memory cards, SmartMedia cards, CompactFlash (CF) cards, Memory Sticks, and/or the like. Further, a non-volatile computer-readable storage medium may also include conductivebridging random access memory (CBRAM), phase-change random access memory (PRAM), ferroelectric random-access memory (FeRAM), non-volatile random-access memory (NVRAM), magnetoresistive random-access memory (MRAM), resistive random-access memory (RRAM), Silicon-Oxide-Nitride-Oxide-Silicon memory (SONOS), floating junction gate random access memory (FJG RAM), Millipede memory, racetrack memory, and/or the like.

A volatile computer-readable storage medium may include random access memory (RAM), dynamic random access memory (DRAM), static random access memory (SRAM), fast page mode dynamic random access memory (FPM DRAM), extended data-out dynamic random access memory (EDO DRAM), synchronous dynamic random access memory (SDRAM), double data rate synchronous dynamic random access memory (DDR SDRAM), double data rate type two synchronous dynamic random access memory (DDR2 SDRAM), double data rate type three synchronous dynamic random access memory (DDR3 SDRAM), Rambus dynamic random access memory (RDRAM), Twin Transistor RAM (TTRAM), Thyristor RAM (T-RAM), Zero-capacitor (Z-RAM), Rambus in-line memory module (RIMM), dual in-line memory module (DIMM), single in-line memory module (SIMM), video random access memory (VRAM), cache memory (including various levels), flash memory, register memory, and/or the like. It will be appreciated that where embodiments are described to use a computer-readable storage medium, other types of computer-readable storage media may be substituted for or used in addition to the computer-readable storage media described above.

As should be appreciated, various embodiments of the present disclosure may also be implemented as methods, apparatus, systems, computing devices, computing entities, and/or the like. As such, embodiments of the present disclosure may take the form of an apparatus, system, computing device, computing entity, and/or the like executing instructions stored on a computer-readable storage medium to perform certain steps or operations. Thus, embodiments of the present disclosure may also take the form of an entirely hardware embodiment, an entirely computer program product embodiment, and/or an embodiment that comprises a combination of computer program products and hardware performing certain steps or operations.

Embodiments of the present disclosure are described below with reference to block diagrams and flowchart illustrations. Thus, it should be understood that each block of the block diagrams and flowchart illustrations may be implemented in the form of a computer program product, an entirely hardware embodiment, a combination of hardware and computer program products, and/or apparatus, systems, computing devices, computing entities, and/or the like carrying out instructions, operations, steps, and similar words used interchangeably (e.g., the executable instructions, instructions for execution, program code, and/or the like) on a computer-readable storage medium for execution. For example, retrieval, loading, and execution of code may be performed sequentially such that one instruction is retrieved, loaded, and executed at a time. In some example embodiments, retrieval, loading, and/or execution may be performed in parallel such that multiple instructions are retrieved, loaded, and/or executed together. Thus, such embodiments may produce specifically configured machines performing the steps or operations specified in the block diagrams and flowchart illustrations. Accordingly, the block diagrams and flowchart illustrations support various combinations of embodiments for performing the specified instructions, operations, or steps.

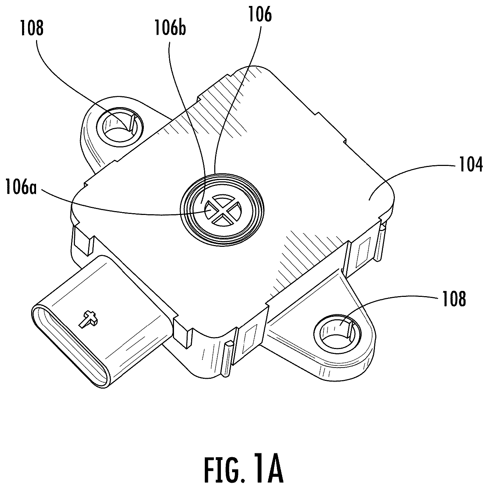

FIG. 1a illustrates an example sensor device 100 in accordance with at least one embodiment of the present disclosure. In particular, FIG. 1a illustrates an example gas sensor device 100 (sensor device 100) configured to detect and measure the concentration level of at least hydrogen gas. In some examples, the sensor device 100 may be configured to perform other functionalities such as, but not limited to pressure sensing, temperature sensing, humidity sensing, and/or the like. For example, the sensor device 100 may comprise one or more sensors such as, but not limited to gas sensor, pressure sensor, temperature sensor, humidity sensor, or the like

In various embodiments, the sensor device 100 is configured to perform sensor drift analysis with respect to one or more sensors of the sensor devices. In some embodiments, the sensor drift analysis includes detecting and/or correcting or otherwise adjusting for sensor drift with respect to the particular sensor. In particular, according to various embodiments of the present disclosure, the sensor device 100 comprises a gas sensor for detecting hydrogen leakage and incorporates the sensor drift adjustment techniques, described herein, configured to detect and correct for sensor drift associated with the gas sensor. In some embodiments, the gas sensor is a thermal conductivity sensor or otherwise operates based on thermal conductivity sensing technology.

The example sensor device 100 may comprise a housing 104 defining a gas port 106. The gas port 106 may be configured to allow gas (e.g., gas, such as hydrogen, to be detected and/or measured by the sensor device 100) to pass into the sensor device 100. The gas port 106 may define one or more openings 106a. In some examples, the gas port 106 may comprise a cover 106b defining the one or more openings 106a.

The housing 104 of the example sensor device 100 may be made of a metal alloy (e.g., stainless steel, carbon steel, or the like) or other suitable material. In some examples, the housing 104 (or a portion of the housing 104) may have a cube shape. It would be understood that the sensor device 100 may have other shapes such as, but not limited to square shape, irregular shape, spherical shape, rectangular shape, round shape, spherical shape, or the like without deviating from the scope of the present disclosure. The housing 104 may house various components of the example sensor device 100, such as, but not limited to, a gas sensor (e.g., a gas sensing element) configured for detecting the presence and/or concentration level of a gas such as hydrogen gas, a controller such as microcontroller unit, and/or sensor drift adjustment module. In some embodiments, the controller may embody the sensor drift adjustment module.

In some examples, the sensor device 100 may comprise one or more mounting members 108 configured for mounting the sensor device 100 to a structure. For example, the one or more mounting members 108 may be leveraged to couple, mount, or otherwise install the sensor device 100 in an area and/or facility to detect hydrogen leakage or other gas. In some examples, as shown in FIG, 1, the one or more mounting members 108 may comprise at least a pair of mounting members 108, each extending outwardly from opposing sides of the sensor device 100. In some examples, a mounting member 108 may define a through hole to facilitate mounting of the sensor device 100 to a structure.

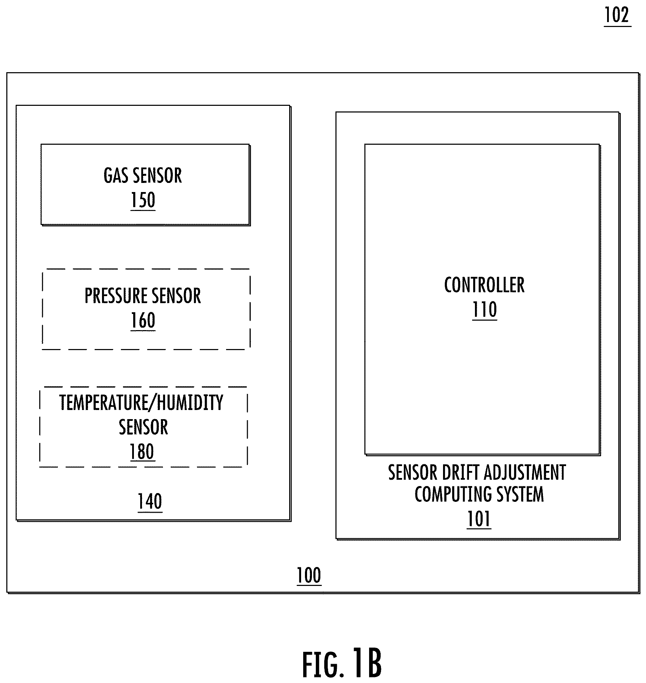

FIG. 1B illustrates a block diagram of an example system architecture 102 for a sensor such as, for example, a sensor device 100 accordance with at least one embodiment of the present disclosure.

The depiction of the example system architecture 102 is not intended to limit or otherwise confine the embodiments described and contemplated herein to any particular configuration of elements or systems, nor is it intended to exclude any alternative configurations or systems for the set of configurations and systems that can be used in connection with embodiments of the present disclosure. Rather, FIG. 1B and the system architecture 102 disclosed therein is merely presented to provide an example basis and context for the facilitation of some of the features, aspects, and uses of the methods, apparatuses, computer readable media, and computer program products disclosed and contemplated herein. It will be understood that while many of the aspects and components presented in FIG. 1B are shown as discrete, separate elements, other configurations may be used in connection with the methods, apparatuses, computer readable media, and computer programs described herein, including configurations that combine, omit, separate, and/or add aspects and/or components. The example system architecture 102 may be used in a plurality of domains and not limited to any specific application as disclosed herewith. The plurality of domains may include healthcare, industrial, manufacturing, education, retail, to name a few.

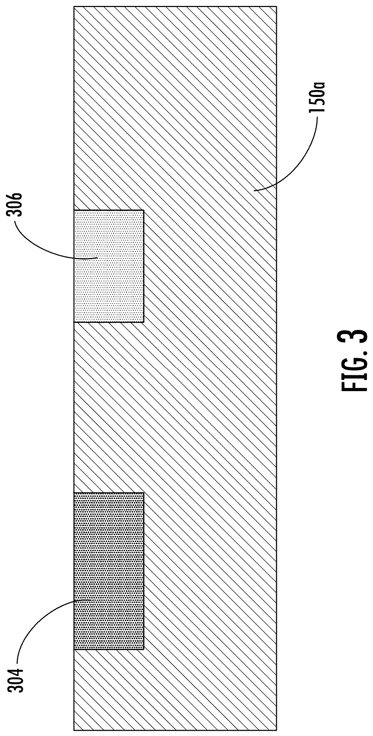

As illustrated, the system architecture 102 includes a sensor drift adjustment system 101 in communication with a sensor system 140. A sensor system 140 may include one or more sensors such as gas sensor 150 (e.g., gas sensing element), pressure sensor 160, and/or temperature sensor 180 (and/or humidity sensor). For example, in some embodiments, the gas sensor 150 may be a gas sensing element, such as described with respect to FIG. 3. In some embodiments, a sensor device 100 may include a computing system 101. In some embodiments, the computing system 101 may include a sensor drift adjustment controller 110 (controller 110).

In some embodiments, the sensor drift adjustment system 101 communicates with the sensor system 140 over one or more communications network(s). As shown in FIG. 1B, in some embodiments, the sensor drift adjustment computing system 101 may be embodied by a sensor device 100. In some embodiments, the sensor drift adjustment computing system 101 may be embodied by the controller 110. In some embodiments, the sensor drift adjustment computing system 101 may embody the controller 110. Alternatively, or additionally, in some embodiments, the sensor drift adjustment computing system 101 may not be embodied by the sensor device 100. In some embodiments, the sensor drift adjustment computing system 101 may be a separate discrete system. In some embodiments, the sensor drift adjustment computing system 101 may embody the sensor device 100.

It should be appreciated that the communications network in some embodiments is embodied in any of a myriad of network configurations. In some embodiments, the communications network embodies a public network (e.g., the Internet). In some embodiments, the communications network embodies a private network (e.g., an internal localized, or closedoff network between particular devices). In some other embodiments, the communications network 105 embodies a hybrid network (e.g., a network enabling internal communications between particular connected devices and external communications with other devices). The communications network in some embodiments includes one or more base station(s), relay(s), router(s), switch(es), cell tower(s), communications cable(s) and/or associated routing station(s), and/or the like. In some embodiments, the communications network includes one or more user-controlled computing device(s) (e.g., a user owned router and/or modem) and/or one or more external utility devices (e.g., Internet service provider communication tower(s) and/or other device(s)).

Each of the components of the system architecture 102 may be communicatively coupled to transmit data to and/or receive data from one another over the same or different wireless and/or wired networks embodying the communications network. Such configuration(s) include, without limitation, a wired or wireless Personal Area Network (PAN), Local Area Network (LAN), Metropolitan Area Network (MAN), Wide Area Network (WAN), and/or the like. Additionally, while FIG. 1B illustrate certain system entities as separate, standalone entities communicating over the communications network, the various embodiments are not limited to this architecture. In other embodiments, one or more computing entities share one or more components, hardware, and/or the like, or otherwise are embodied by a single computing device such that connection(s) between the computing entities are over the communications network are altered and/or rendered unnecessary. For example, in some embodiments, a sensor system 140 may include some or all of the sensor drift adjustment system 101, such that an external communications network 105 is not required.

In some embodiments, the sensor system 140 (or portion thereof) and the sensor drift adjustment system 101 are embodied in an on-premises system within or associated with a building. In some such embodiments, the sensor system 140 (or portion thereof) and the sensor drift adjustment system 101 are communicatively coupled via at least one wired connection. Alternatively, or additionally, in some embodiments, the sensor system 140 embodies or includes the sensor drift adjustment system 101 (or portion thereof), for example as a software component.

The sensor drift adjustment system 101 may include any number of computing devices and/or systems configured to control one or more functionalities provided by the sensor system 140 in accordance with techniques described herein. As shown in FIG. 1B, in some embodiments, the sensor drift adjustment system 101 may comprise a controller 210. In some embodiments, the sensor drift adjustment system 101 is configured to detect and adjust sensor drift associated with the gas sensor 150.

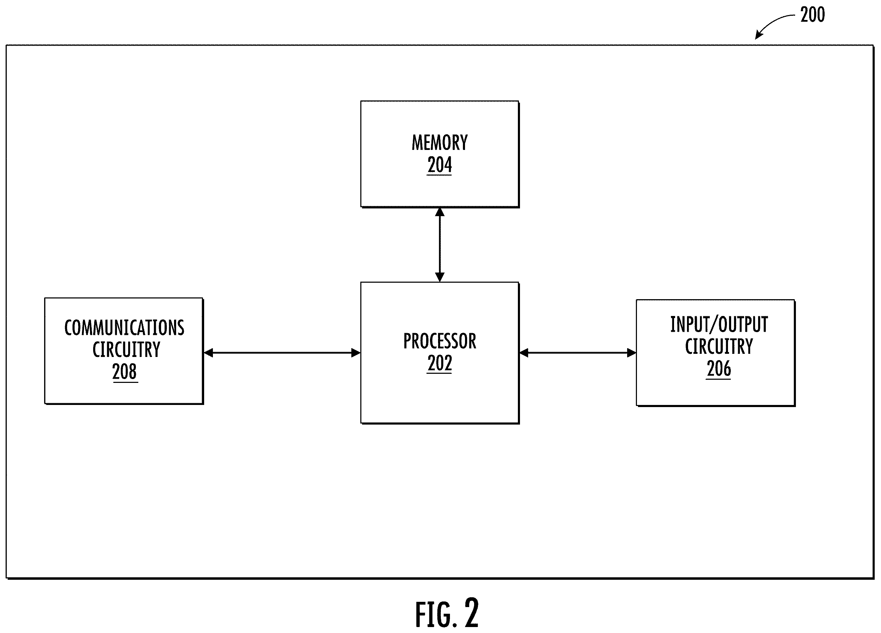

FIG. 2 illustrates a block diagram of an example apparatus that may be specially configured in accordance with at least one example embodiment of the present disclosure. Specifically, FIG. 2 depicts an example sensor drift adjustment apparatus 200 ("apparatus 200") specially configured in accordance with at least some example embodiments of the present disclosure. In some embodiments, the sensor drift adjustment system 101 and/or a portion thereof is embodied by one or more system(s), such as the apparatus 200 as depicted and described in FIG. 2. The apparatus 200 includes processor 202, memory 204, input/output circuitry 206, and/or communications circuitry 208. In some embodiments, the apparatus 200 is configured, using one or more of the sets of circuitry embodied by processor 202, memory 204, input/output circuitry 206, and/or communications circuitry 208 to execute and perform the operations described herein.

In general, the terms computing entity (or "entity" in reference other than to a user), device, system, and/or similar words used herein interchangeably may refer to, for example, one or more computers, computing entities, desktop computers, mobile phones, tablets, phablets, notebooks, laptops, distributed systems, items/devices, terminals, servers or server networks, blades, gateways, switches, processing devices, processing entities, set-top boxes, relays, routers, network access points, base stations, the like, and/or any combination of devices or entities adapted to perform the functions, operations, and/or processes described herein. Such functions, operations, and/or processes may include, for example, transmitting, receiving, operating on, processing, displaying, storing, determining, creating/generating, monitoring, evaluating, comparing, and/or similar terms used herein interchangeably. In one embodiment, these functions, operations, and/or processes can be performed on data, content, information, and/or similar terms used herein interchangeably. In this regard, the apparatus 200 embodies a particular, specially configured computing entity transformed to enable the specific operations described herein and provide the specific advantages associated therewith, as described herein.

Although components are described with respect to functional limitations, it should be understood that the particular implementations necessarily include the use of particular computing hardware. It should also be understood that in some embodiments certain of the components described herein include similar or common hardware. For example, in some embodiments two sets of circuitry both leverage use of the same processor(s), network interface(s), storage medium(s), and/or the like, to perform their associated functions, such that duplicate hardware is not required for each set of circuitry. The use of the term "circuitry" as used herein with respect to components of the apparatuses described herein should therefore be understood to include particular hardware configured to perform the functions associated with the particular circuitry as described herein.

Particularly, the term "circuitry" should be understood broadly to include hardware and, in some embodiments, software for configuring the hardware. For example, in some embodiments, "circuitry" includes processing circuitry, storage media, network interfaces, input/output devices, and/or the like. Alternatively, or additionally, in some embodiments, other elements of the apparatus 200 provide or supplement the functionality of another particular set of circuitry. For example, the processor 202 in some embodiments provides processing functionality to any of the sets of circuitry, the memory 204 provides storage functionality to any of the sets of circuitry, the communications circuitry 208 provides network interface functionality to any of the sets of circuitry, and/or the like.

In some embodiments, the processor 202 (and/or co-processor or any other processing circuitry assisting or otherwise associated with the processor) is/are in communication with the memory 204 via a bus for passing information among components of the apparatus 200. In some embodiments, for example, the memory 204 is non-transitory and may include, for example, one or more volatile and/or non-volatile memories. In other words, for example, the memory 204 in some embodiments includes or embodies an electronic storage device (e.g., a computer readable storage medium). In some embodiments, the memory 204 is configured to store information, data, content, applications, instructions, or the like, for enabling the apparatus 200 to carry out various functions in accordance with example embodiments of the present disclosure.

The processor 202 may be embodied in a number of different ways. For example, in some example embodiments, the processor 202 includes one or more processing devices configured to perform independently. Additionally, or alternatively, in some embodiments, the processor 202 includes one or more processor(s) configured in tandem via a bus to enable independent execution of instructions, pipelining, and/or multithreading. The use of the terms "processor" and "processing circuitry" should be understood to include a single core processor, a multi-core processor, multiple processors internal to the apparatus 200, and/or one or more remote or "cloud" processor(s) external to the apparatus 200.

In an example embodiment, the processor 202 is configured to execute instructions stored in the memory 204 or otherwise accessible to the processor. Alternatively, or additionally, the processor 202 in some embodiments is configured to execute hard-coded functionality. As such, whether configured by hardware or software methods, or by a combination thereof, the processor 202 represents an entity (e.g., physically embodied in circuitry) capable of performing operations according to an embodiment of the present disclosure while configured accordingly. Alternatively, or additionally, as another example in some example embodiments, when the processor 202 is embodied as an executor of software instructions, the instructions specifically configure the processor 202 to perform the algorithms embodied in the specific operations described herein when such instructions are executed. As one particular example embodiment, the processor 202 is configured to perform various operations associated with performing improved asset monitoring associated with a process control and automation system.

In some embodiments, the apparatus 200 includes input/output circuitry 206 that provides output to the user and, in some embodiments, to receive an indication of a user input. In some embodiments, the input/output circuitry 206 is in communication with the processor 202 to provide such functionality. The input/output circuitry 206 may comprise one or more user interface(s) and in some embodiments includes a display that comprises the interface(s) rendered as a web user interface, an application user interface, a user device, a backend system, or the like. In some embodiments, the input/output circuitry 206 also includes a keyboard, a mouse, a joystick, a touch screen, touch areas, soft keys a microphone, a speaker, or other input/output mechanisms. The processor 202 and/or input/output circuitry 206 comprising the processor may be configured to control one or more functions of one or more user interface elements through computer program instructions (e.g., software and/or firmware) stored on a memory accessible to the processor (e.g., memory 204, and/or the like). In some embodiments, the input/output circuitry 206 includes or utilizes a user-facing application to provide input/output functionality to a client device and/or other display associated with a user.

In some embodiments, the apparatus 200 includes communications circuitry 208. The communications circuitry 208 includes any means such as a device or circuitry embodied in either hardware or a combination of hardware and software that is configured to receive and/or transmit data from/to a network and/or any other device, circuitry, or module in communication with the apparatus 200. In this regard, in some embodiments the communications circuitry 208 includes, for example, a network interface for enabling communications with a wired or wireless communications network. Additionally or alternatively in some embodiments, the communications circuitry 208 includes one or more network interface card(s), antenna(s), bus(es), switch(es), router(s), modem(s), and supporting hardware, firmware, and/or software, or any other device suitable for enabling communications via one or more communications network(s). Additionally, or alternatively, the communications circuitry 208 includes circuitry for interacting with the antenna(s) and/or other hardware or software to cause transmission of signals via the antenna(s) or to handle receipt of signals received via the antenna(s). In some embodiments, the communications circuitry 208 enables transmission to and/or receipt of data from user device, one or more asset(s) or accompanying sensor(s), and/or other external computing device in communication with the apparatus 200.

In some embodiments, two or more of the sets of circuitries 202-208 are combinable. Alternatively, or additionally or in some embodiments, one or more of the sets of circuitries embodying processor 202, memory 204, input/output circuitry 206, and/or communications circuitry 208, perform some or all of the functionality described as associated with another component. For example, in some embodiments, two or more of the sets of circuitries embodied by processor 202, memory 204, input/output circuitry 206, and/or communications circuitry 208 are combined into a single module embodied in hardware, software, firmware, and/or a combination thereof.

FIG. 3 illustrates a cross-section view of an example cross section of a thermal conductive sense element 150a in accordance with at least one embodiments of the present disclosure. As described above, the gas sensor 150 may comprise a thermal conductive sense element that functions based on the principles of thermal conductivity sensor technology. The gas sensor 150 may be configured to detect heat loss on the IC (I.e., the sensing element). As shown in FIG. 3, the thermal conductive sense element 150a may comprise a heater 306 disposed thereof. The heater 306 may be configured to turn on and off on a periodic basis. The heater 306 provides heat that is detected by the thermopile 304, which is a network of conductors. The thermopile 304 provides/generates an electrical signal based on the temperature it receives from the heater 306, which may be proportional to the temperature differential. When there is heat loss, conductive heat transfers to the ambient gas, and because there is a high hydrogen content in the air, the thermopile 304 will be cooler than normal which is translated into Hydrogen PPM output. In this regard, the lower the temperature of the thermopile 304, the higher the concentration of hydrogen in the surrounding air.

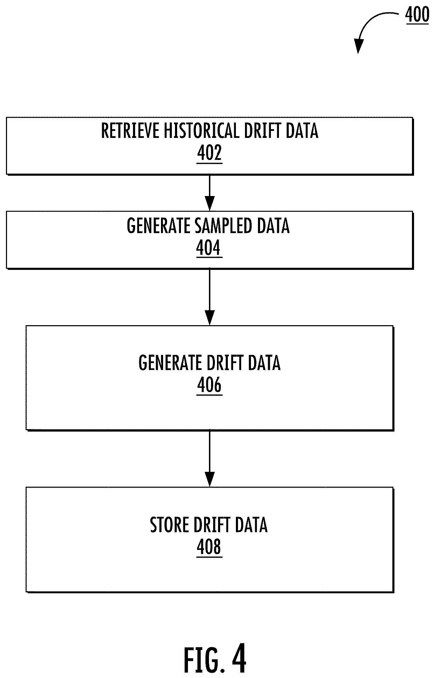

FIG. 4 illustrates an example flow chart diagram of an example process 400 for sensor drift adjustment in accordance with at least one embodiment of the present disclosure. As described above, many factors may affect the accuracies of gas sensor readings, which may result in sensor drift. In some embodiments, a sensor drift is an erroneous positive or negative reading from a gas sensor such as a hydrogen sensor for detecting hydrogen leakage. For example, a sensor drift may suggest the presence of the target gas to be detected (e.g., hydrogen or the like) when the target gas in not present. The flow chart diagram of the example process 400 depicts an example automated and robust sensor drift adjustment that overcome the various technical challenges associated with sensor drift and improve the accuracies in sensor readings by, for example, performing periodic analysis to detect and correct sensor drift.

Although the example processes depicts a particular sequence of operations, the sequence may be altered without departing from the scope of the present disclosure. For example, some of the operations depicted may be performed in parallel or in a different sequence that does not materially affect the function of the processes. The blocks indicate operations of each process. Such operations may be performed in any of a number of ways, including, without limitation, in the order and manner as depicted and described herein. In some embodiments, one or more blocks of any of the processes described herein occur inbetween one or more blocks of another process, before one or more blocks of another process, in parallel with one or more blocks of another process, and/or as a sub-process of a second process. Additionally or alternatively, any of the processes in various embodiments include some or all operational steps described and/or depicted, including one or more optional blocks in some embodiments. With regard to the flowcharts illustrated herein, one or more of the depicted block(s) in some embodiments is/are optional in some, or all, embodiments of the disclosure. Optional blocks are depicted with broken (or "dashed") lines. Similarly, it should be appreciated that one or more of the operations of each flowchart may be combinable, replaceable, and/or otherwise altered as described herein.

The process 400 may be implemented by one or more computing devices, entities, and/or systems described herein. For example, via, the various steps/operations of the process 400, the computing system 101 described herein (or portion thereof) may perform periodic analysis to detect, calculate, and/or correct sensor drift. In some examples, each block/step/operation of the flowchart, and combinations of blocks/steps/operations in the flowchart, may be implemented by various means such as hardware, firmware, circuitry and/or other devices associated with execution of software including one or more computer program instructions. In some examples, the process 400 may be performed by a processing circuitry (for example, but not limited to, an application-specific integrated circuit (ASIC), a central processing unit (CPU)), a microcontroller (MCU). In some examples, the processing circuitry may be electrically coupled to and/or in electronic communication with other circuitries of the example sensor device 100.

In some embodiments, the process 400 is embodied by computer program code stored on a non-transitory computer-readable storage medium of a computer program product configured for execution to perform the process 400 as depicted and described. Alternatively or additionally, in some embodiments, the process 400 is performed by one or more specially configured computing devices, such as the apparatus 200 alone or in communication with one or more other component(s), device(s), system(s), and/or the like. In this regard, in some such embodiments, the apparatus 200 is specially configured by computer-coded instructions (e.g., computer program instructions) stored thereon, for example in the memory 204 and/or another component depicted and/or described herein and/or otherwise accessible to the apparatus 200, for performing the operations as depicted and described. In some embodiments, the apparatus 200 is in communication with one or more external apparatus(es), system(s), device(s), and/or the like, to perform one or more of the operations as depicted and described. For example, the apparatus 200 in some embodiments is in communication with separate component(s) of a network, external network(s), and/or the like, to perform one or more of the operation(s) as depicted and described. For purposes of simplifying the description, the process 400 is described as performed by and from the perspective of the apparatus 200.

In some embodiments, the process 400 comprises, at step/operation 402, retrieving historical drift data. For example, the computing system 101 (or portion thereof) may retrieve the historical drift data in response to indication of initiation of a runtime (e.g., start of a runtime). In some embodiments, a runtime refers to when the sensor device 100 is powered on. In some embodiments, the computing system 101 may detect or receive indication of a runtime based on an internal clock associated with the sensor device 100. The computing system 101, using the internal clock, may be configured to determine when the sensor device 100 transitions to an operation mode (e.g., when powered on) and track the runtime hours. For example, the computing system 101, using the internal clock, may be configured to start counting up the hours that the sensor device is operating.

In some embodiments, retrieving the historical drift data comprises reading the immediately preceding drift data stored in a memory associated with the sensor device 100 and/or the computing system 101 and/or the. In some embodiments, the historical data comprises the output of a previous drift adjustment process. As described herein, the computing system 101 may store the calculated drift data output (e.g., Hydrogen PPM) collected in the previous run (e.g., previous execution of the drift adjustment process) in a memory such as a non-volatile memory.

For example, the computing system 101 may be configured to read the last drift data stored in the memory, which corresponds to the drift data for the previous execution of the drift correction process according to techniques of the present disclosure. In some embodiments, the computing system leverages the historical drift data as a reference point to make a determination if the sensor device 100 has drifted since the previous drift data calculation.

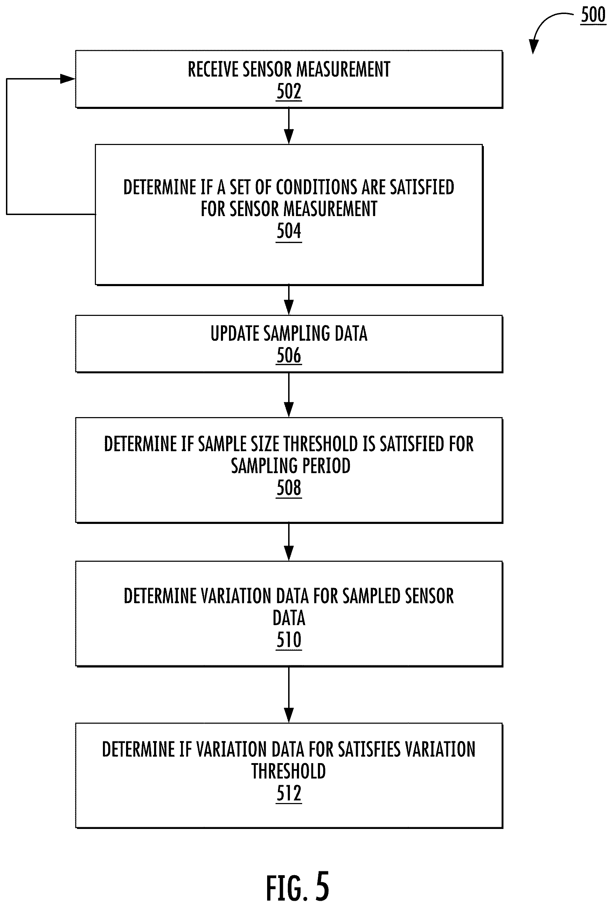

In some embodiments, the process 400 comprises, at step/operation 404 generating sampled sensor data for the current sampling period (e.g., sampling interval). In some embodiments, the sampled sensor data comprises sensor measurements that satisfy one or more conditions, thresholds, and/or the like as described herein. In some embodiments, the sampled sensor data are representative and/or indicative of drift measurement. In some embodiments, the sampled sensor data may be generated in accordance with the process 500 illustrated in FIG. 5, which is a process for generating sampled sensor data for draft calculation in accordance with at least some example embodiments of the present disclosure.

In some embodiments, the process 500 comprises, at step/operation 502 receiving sensor measurements at predetermined intervals. For example, the computing system 101 may receive sensor measurements from the gas sensor 150. In some embodiments, receiving the sensor measurement comprise receiving a plurality of sensor measurements. In some embodiments, each sensor measurement of the plurality of sensor measurements is received at a predetermined interval.

In an example implementation, the predetermined interval is every second. For example, in some implementations, the computing system 101 may be configured to receive sensor output every second. It would be appreciated that the predetermined intervals may be less or greater than every second and/or may be at unequal intervals such as, for example, every two seconds, every five minutes, every 2 seconds for a first duration and every 4 seconds for a second duration within the same sampling period.

In some embodiments, the process 500 comprises, at step/operation 504, determining if a set of operational conditions is satisfied for a sensor measurement. For example, the computing system 101 may determine if a set of operational conditions is satisfied by comparing the corresponding current operational data to the set of operational conditions. In some embodiments, the set of operational conditions comprise a temperature range or threshold, a humidity range or threshold, and/or a pressure stability duration. In an example implementation, the temperature range is 20°C to 30°C, In an example implementation, the humidity range is 10% to 60% rH. In an example implementation, the pressure stability duration is 1 minute. For example, in such example implementation, the set of operational conditions may comprise temperature of about 25°C; humidity of about 50% rH, and/or pressure level of about 1 bar. It would be appreciated that the set of operational conditions and/or the corresponding ranges, threshold, duration, and/or the like may be different without deviating from the scope of the present disclosure.

In some embodiments, the set of operational conditions refer to conditions for determining if a sensor measurement (e.g., a data point such as a single hydrogen ppm output by the gas sensor 150) should be used or discarded in the drift calculation. For example, the output of a gas sensor 150 may vary over temperature, humidity, and/or pressure. By aggregating sensor data that meet the set of operational conditions, example embodiments, minimize or eliminate the influence of variation of these operating parameters, resulting in more accurate sensor drift calculation and correction. In some embodiments, determining if the set of operational conditions is satisfied comprises determining the current operational data (e.g., temperature data, pressure data, humidity data, and/or the like). In some embodiments, determining the current operational data may comprise receiving the current operational data from one or more sensors (e.g., temperature sensors, humidity sensor, pressure sensor, and/or the like) which may be embodied by the sensor device 100.

In some embodiments, the process 500 comprises, at step/operation 506, updating the sampled sensor data for the sampling period to include the sensor measurement in response to determining that the current operational condition associated with the sensor measurement (e.g., the temperature, pressure, humidity, and/or the like) at the time the gas sensor measured the gas concentration (such as, for example, hydrogen concentration) satisfies the set of operational conditions. In some embodiments, the sampled sensor data comprises the sensor measurements (e.g., sensor output) for a sampling period that at least satisfy the set of operational conditions. In some embodiments, the step/operation 506 comprise discarding the sensor measurement in response to determining that the current operational condition associated with the sensor measurement fails to satisfy the set of operational conditions.

In some embodiments, the process 500 comprises, at step/operation 508, determining if the sampled sensor data for the sampling period satisfies a sample size threshold. For example, the computing system 101 may analyze the sampled sensor data after the sampling period has elapsed to determine if the sample size (e.g., number of sensor measurements) in the sampled sensor data satisfies the sample size threshold. In this regard, in some embodiments, the sampled sensor data after process 500 may comprise at least a subset of the sensor measurements collected by the computing system 101 that satisfy a set of operational conditions and a sample size threshold. In an example implementation the sample size is 100 sensor outputs (e.g., 100 sensor measurements) and the sampling period is six minutes. It would be appreciated that the sample size threshold and sampling period may be different from the above examples without deviating from the scope of the present disclosure.

As described above, in some embodiments, the sensor measurement (e.g., PPM drift measurement) is taken at sampling intervals, such as every second or the like. In some embodiments, the computing system 101 collects, aggregates, obtains, or the like, the sensor measurements for up to the end of the sampling period or until the sample size threshold is satisfied. For example, the computing system 101 may be configured to terminate collecting sensor data for a sampling period when the sample size threshold has been satisfied even though the sampling period has not been elapsed. In some embodiments, in response to determining that the sampling period has been elapsed, the computing system 101 determines if the sample size threshold is satisfied (e.g., that there is sufficient amount of sensor measurements). For example, a condition where the sample size threshold is not satisfied at the end of the sampling period may be indicative of excessive variation, such that the sampled sensor measurements may not be reliable. In some embodiments, the computing system 101 is configured to deem the sampled sensor data as not usable in response to determining that the sample size is not satisfied.

In some embodiments, the process 500 comprises, at step/operation 510, generating variation data for the sampled sensor data. For example, the computing system 101 may generate the variation data for the sampled sensor data by calculating the variance within the sampled sensor data. In some examples, the computing system 101 may generate the variation data for the sampled sensor data by calculating the standard deviation within the sampled sensor data. In some embodiments, generating the variation data for the sampling period comprises applying one or more analytical models to the sampled sensor data. In some embodiments, the one or more analytical models may define or otherwise comprise a statistical algorithm which may include standard deviation calculation, variance calculation, and/or other statistical measures.

In some embodiments, the process 500 comprises, at step/operation 512, determining if the variation data satisfies a variation threshold. For example, the computing system 101 may compare the variation data for the sampled sensor data to a variance threshold to determine if the variance threshold is satisfied. In an example embodiment, the variance threshold is 75PPM and is satisfied if the variation data for the sampled sensor data is less than the variation threshold.

Returning to FIG. 4, in some embodiments, the process 400 comprises, at step/operation 406, generating the drift data (e.g., predicted drift data). The drift data, for example, may be an estimated drift data determined according to techniques described herein. For example, the computing system 101 may calculate the drift data based at least in part on the sampled sensor data. In some embodiments, generating the drift data comprises arranging the sensor measurements in the sampled sensor data sequentially based on the measurement timestamp associated with each sensor measurement (e.g., when outputted/measured by the gas sensor 150).

For example, in some embodiments, when the sensor device 100 is in an ON state (e.g., at power on) and in response to a successful drift measurement (e.g., in response to determining that the sampled sensor data for the sampling period satisfies the conditions, thresholds, and/or other criteria as described above, the computing system 101 calculates the estimated drift data (e.g., estimated drift) using the sampled sensor data (e.g., the plurality of sensor measurements therein). In some embodiments, calculating the estimated drift data as described above may include observing sequential changes in sensor measurements (Vn, Vn-1, Vn-2, ... Vn-m), where V represents the sensor measurement (e.g., measurement value output by the gas sensor device 100). For example, (i) "Vn < Vn-1" may be indicative that the current measurement Vn is less than the last measurement Vn-1; (ii) "Vn-1 < Vn-2" may be indicative that the last measurement Vn-1 is less than the measurement Vn-2 before that, and so on for the sampling period. For example, n may be indicative of the measurement timestamp of otherwise determined based on the measurement time stamp.

Additionally, or alternatively, in some embodiments, generating the drift data comprises applying a best fit model to the sensor measurements and associated measurement timestamp to generate a slope of linear best fit line (e.g., determining best fit on recent history using the measurement values (Vn) and the timestamps (Tn). For example, the computing system 101 may calculate slope of linear best fit line (e.g., f(V, T)).

Additionally, or alternatively, in some embodiments, generating the drift data comprises calculating the drift data based on observed drift data and one or more drift constraints. In some embodiments, the one or more drift constraints comprises a drift range constraint and a long-term drift constraint. For example, in some embodiments, generating the drift data may comprise calculating the adjustment data based on the observed drift data and the one or more drift constraints (or at least based on the drift range constraint).

In an example implementation, the drift range constraint is between "0" and "-10" ppm. For example, the computing system 101 may calculate the adjustment data of between "0" and "-10" ppm drift from the observed drift data. In some embodiments, the observed drift data is the average of the sensor measurements in the sampled sensor data. The computing system 101 may be configured to determine if the observed drift data goes beyond +0 or to - 10. For example, the computing system 101 may be configured to ensure the drift is within the drift range (e.g., doesn't go beyond +0 to -10).

As described above, in some examples, the one or more drift constraints comprises a drift range and/or a long-term drift constraint. In some embodiments, in response to a successful drift measurement and drift calculation, the one or more drift constraints (or the long-term drift constraint thereof) may be applied. In some embodiments, applying the long-term drift constraint constraints comprises restricting the drift from exceeding the long-term drift constraint, which may be a global standard. In some embodiments, restricting the drift from exceeding the long-term drift comprises comparing the observed drift data for the sampling period to the long-term drift constraint. In some embodiments, if the observed drift data is less than the long-term drift constraint, the observed drift data is selected or otherwise used as the drift data. In some embodiments, if the observed drift data is greater than the long-term drift constraint, the computing system 101 may select or otherwise use the long-term drift constraint. In an example implementation, the long-term drift constraint is 0.3 ppm per hour for the first thousand hours of operation of the sensor device 100 and/or 0.2 ppm per hour after the first thousand hours the drift limit range changes. In this regard, the total drift for the total life of the product may be 3500 ppm. In this regard, the computing system 101 may be configured to adjust the drift at the predetermined sampling period (e.g., six minutes in some examples) while satisfying the one or more drift constraints.

In some embodiments, applying the one or more drift constraints comprises restricting the drift from exceeding 0 ppm output compensation. For example, if the drift adjustment caused output to exceed 0ppm, the computing system 101 may reduce the drift value to cause 0ppm output. Further, if the drift adjustment causes output to be less than 0ppm, the computing system 101 may use the drift data (e.g., observed drift data) value as calculated with no change.

In some embodiments, a drift variable is calculated. The drift variable may refer to the selected drift data or constraint based on the constraints above. The drift variable for example may refer to the amount of drift and may be stored in a variable and used to adjust the subsequent sensor outputs. In some embodiments, the drift variable may be used to adjust subsequent outputs based on the equation: Reported Output = calculated output - drift variable, which may be in ppm. In some examples the drift variable may be: zero at startup. During initialization, if drift samples are recorded, the computing system 101 may perform drift calculation to update the drift variable. The system 101 may start a request for drift sampling to trigger drift calculation and update. In some examples, upon generating a new drift sample (e.g., new sampled data for a second/another sampling period), the system 101, may perform drift calculation on the new sample, constrain the drift based on average ppm value, and update the drift variable.

In some embodiments, the process 400 comprises, at step/operation 408, storing the drift data. For example, the computing system 101 may store the drift data in memory associated with the sensor device 100. In some embodiments, the computing system 101 is configured to adjust subsequent sensor device output (e.g., sensor measurement) by applying a drift adjustment model to the device sensor device output. In an example implementation, the drift adjustment model may define the equation: Adjusted output (e.g., reported output) = sensor device output - current drift data. In some embodiments, the drift adjustment model may be generated based on the stored estimated drift data.

### CONCLUSION

Many modifications and other embodiments of the disclosure set forth herein will come to mind to one skilled in the art to which this disclosure pertains having the benefit of the teachings presented in the foregoing description and the associated drawings. Therefore, it is to be understood that the embodiments are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

While this specification contains many specific implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single product or packaged into multiple products.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

Further, while this detailed description has set forth some embodiments of the present disclosure, the appended claims may cover other embodiments of the present disclosure which differ from the described embodiments according to various modifications and improvements.
Further, within the appended claims, unless the specific terms "means for" or "step for" is used within a given claim, it is not intended that the claim be interpreted under 35 U.S.C. § 112, paragraph (f).

## Claims

1. A computer-implemented method comprising:
receiving a plurality of sensor measurements from a gas sensor for at least a portion of a sampling period;
generating sampled sensor data from the plurality of sensor measurements, wherein the sampled sensor data comprises at least a subset of the plurality of sensor measurements that satisfy a set of operational conditions and a sample size threshold;
generating estimated drift data based on the sampled sensor data and one or more drift constraints;
storing the estimated drift data; and
adjusting gas sensor output by applying a drift adjustment model to the gas sensor output, wherein the drift adjustment model is generated based on the stored estimated drift data.

2. The computer-implemented method of claim 1, wherein generating the sampled sensor data further comprises:
generating variation data for the sampling period by applying one or more analytical models to the sampled sensor data; and
determining if the variation data satisfies a variation threshold.

3. The computer-implemented method of claim 1, wherein the set of operational conditions comprise one or more of a temperature range, a humidity range, or a pressure stability duration.

4. The computer-implemented method of claim 3, further comprising:
receiving, from the gas sensor (i) temperature data, (ii) humidity data, and (iii) pressure data associated with a sensor measurement from the plurality of sensor measurements; and
determining if (i) the temperature data satisfies a temperature threshold, (ii) the humidity data satisfies a humidity threshold; and (iii) the pressure data satisfies a pressure threshold.

5. The computer-implemented method of claim 4, wherein the gas sensor is configured for measuring hydrogen gas concentration in air.

6. The computer-implemented method of claim 1, wherein generating the estimated drift data further comprises generating the estimated drift data based on one or more sensor output compensation constraints.

7. The computer-implemented method of claim 1, wherein receiving the plurality of sensor measurements comprises receiving each sensor measurement of the plurality of sensor measurements at a predetermined interval.

8. An apparatus comprising at least one processor and at least one memory storing instructions that, when executed by the at least one processor, cause the apparatus to:
receive a plurality of sensor measurements from a gas sensor for at least a portion of a sampling period;
generate sampled sensor data from the plurality of sensor measurements, wherein the sampled sensor data comprises at least a subset of the plurality of sensor measurements that satisfy a set of operational conditions and a sample size threshold;
generate estimated drift data based on the sampled sensor data and one or more drift rate constraints;
store the estimated drift data; and
adjust subsequent gas sensor output by applying a drift adjustment model to the gas sensor output, wherein the drift adjustment model is generated based on the stored estimated drift data.

9. The apparatus of claim 8, wherein generating the sampled sensor data further comprises:
generating variation data for the sampling period by applying one or more analytical models to the sampled sensor data; and
determining if the variation data satisfies a variation threshold.

10. The apparatus of claim 8, wherein the set of operational conditions comprise one or more of a temperature range, a humidity range, or a pressure stability duration.

11. The apparatus of claim 10, further comprising:
receiving, from the gas sensor (i) temperature data, (ii) humidity data, and (iii) pressure data associated with a sensor measurement from the plurality of sensor measurements; and
determining if (i) the temperature data satisfies a temperature threshold, (ii) the humidity data satisfies a humidity threshold; and (iii) the pressure data satisfies a pressure threshold.

12. The apparatus of claim 11, wherein the gas sensor is configured for measuring hydrogen gas concentration in air.

13. The apparatus of claim 8, wherein generating the estimated drift data further comprises generating the estimated drift data based one or more sensor output compensation constraints.

14. The apparatus of claim 8, wherein receiving the plurality of sensor measurements comprises receiving each sensor measurement of the plurality of sensor measurements at a predetermined interval.

15. At least one non-transitory computer-readable storage medium having computer coded instructions configured to, when executed by at least one processor:
receive a plurality of sensor measurements from a gas sensor for at least a portion of a sampling period;
generate sampled sensor data from the plurality of sensor measurements, wherein the sampled sensor data comprises at least a subset of the plurality of sensor measurements that satisfy a set of operational conditions and a sample size threshold;
generate estimated drift data based on the sampled sensor data and one or more drift rate constraints;
store the estimated drift data; and
adjust subsequent gas sensor output by applying a drift adjustment model to the gas sensor output, wherein the drift adjustment model is generated based on the stored estimated drift data.
